(19) Europäisches Patentamt
European Patent Office
Office européen des brevets

(11) **EP 3 078 330 A1**

(12) **EUROPEAN PATENT APPLICATION**
published in accordance with Art. 153(4) EPC

(43) Date of publication:
**12.10.2016 Bulletin 2016/41**

(51) Int Cl.:
**A61B 8/08** *(2006.01)*

(21) Application number: **14867120.9**

(86) International application number:
**PCT/JP2014/079163**

(22) Date of filing: **04.11.2014**

(87) International publication number:
**WO 2015/083471 (11.06.2015 Gazette 2015/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.12.2013 JP 2013252224**

(71) Applicant: **Olympus Corporation**
**Tokyo 192-8507 (JP)**

(72) Inventor: **EDA, Hirotaka**
**Tokyo 192-8507 (JP)**

(74) Representative: **Gunzelmann, Rainer**
**Wuesthoff & Wuesthoff**
**Patentanwälte PartG mbB**
**Schweigerstraße 2**
**81541 München (DE)**

(54) **ULTRASONIC OBSERVATION DEVICE, ULTRASONIC OBSERVATION DEVICE OPERATION METHOD, AND ULTRASONIC OBSERVATION DEVICE OPERATION PROGRAM**

(57) An ultrasonic observation apparatus includes a storage unit for storing a plurality of data sets including position information indicating a position of an observation point, the feature extracted by a computation unit for the observation point, image data generated by an image processing unit for the observation point, and parameters used for extracting the feature and generating the image data, a data selection unit for searching the data sets based on relative position information indicating a relative position between a patient and an ultrasound probe and selecting a data set meeting a predetermined condition, and an execution control unit for, when a data set meeting the predetermined condition is selected, causing the computation unit to re-extract the feature by use of the parameters included in the data set.

FIG.1

**Description**

Field

The present invention relates to an ultrasonic observation apparatus for observing tissues of a specimen by use of ultrasonic waves, a method for operating the ultrasonic observation apparatus, and a program for operating the ultrasonic observation apparatus.

Background

Conventionally, there has been known a diagnosis technique using past examination results. For example,
[0003] Patent Literature 1 discloses a medical image display system for overlapping and displaying past additional information on a captured image to be diagnosed, or overlapping and displaying a past-captured image and additional information corresponding thereto on a display means, and then switching and displaying only the captured image to a latest captured image.
Patent Literature 2 discloses a diagnosis support apparatus for detecting a temporal change in a feature parameter of the same tumor from a plurality of medical images acquired for the same patient at different times and correcting a determination result as to whether the tumor is benign or malignant based on the temporal change.

Citation List

Patent Literature

Patent Literature 1: Japanese Laid-open Patent Publication No. 2008-6169
Patent Literature 2: Japanese Laid-open Patent Publication No. 2005-323629

Summary

Technical Problem

In Patent Literature 1, however, the additional information on the past-captured image extracted based on patient information is only overlapped and displayed on the image displayed on the screen, and thus an operator cannot directly grasp a temporal change in the site under observation.
Further, in Patent Literature 2, specific sites are extracted from a plurality of already-acquired images, respectively, and feature parameters of the specific sites are calculated, respectively, and thus an operator cannot directly grasp temporal changes in the sites under observation.
The present invention has been made in view of the foregoing, and an object of the invention is to provide an ultrasonic observation apparatus enabling a user to directly grasp a temporal change in a site under observation, a method for operating the ultrasonic observation apparatus, and a program for operating the ultrasonic observation apparatus.

Solution to Problem

In order to solve the above described problems and achieve the object, an ultrasonic observation apparatus according to the invention transmits an ultrasonic wave toward a specimen and receives the ultrasonic wave reflected from the specimen by an ultrasound probe to generate an image based on the received ultrasonic wave. The ultrasonic observation apparatus includes: a computation unit configured to extract a feature of the received ultrasonic wave; an image processing unit configured to generate image data based on the received ultrasonic wave; a position information acquisition unit configured to acquire relative position information indicating a relative position of the ultrasound probe relative to a patient as an examination target; a storage unit configured to store a plurality of data sets including: position information on an observation point; the feature extracted by the computation unit for the observation point; the image data generated by the image processing unit for the observation point; and parameters used for extracting the feature and generating the image data such that the plurality of data sets associates with information for identifying the patient; a data selection unit configured to search the plurality of data sets based on the relative position information acquired by the position information acquisition unit, and to select a data set meeting a predetermined condition; and an execution control unit configured to, when the data selection unit selects the data set meeting the predetermined condition, cause the computation unit to re-extract one of a feature included in the data set selected by the data selection unit and a latest feature extracted by the computation unit by use of the parameters used for extracting the other of the features.

In the above-mentioned ultrasonic observation apparatus, the predetermined condition indicates that a position of the observation point presented by the position information included in the data set is closest to the relative position of the ultrasound probe relative to the patient and within a predetermined range of the relative position.

The above-mentioned ultrasonic observation further includes a notification unit configured to, when the data selection unit selects the data set meeting the predetermined condition, give notice that the data set is selected.

In the above-mentioned ultrasonic observation apparatus, the notification unit is a display unit configured to display on a screen an image based on the image data included in the data set selected by the data selection unit.

In the above-mentioned ultrasonic observation, the notification unit is configured to give notice that the data set is selected, by voice, notification sound or text display.

The above-mentioned ultrasonic observation apparatus further includes an input unit configured to input a signal depending on an operation from outside into the execution control unit. The execution control unit is configured to cause the computation unit to start re-extracting the one of the features depending on a predetermined signal input from the input unit after giving notice that the data set is selected.

In the above-mentioned ultrasonic observation apparatus, the execution control unit is configured to cause the computation unit to start re-extracting the one of the features when the data selection unit selects the data set meeting the predetermined condition.

In the above-mentioned ultrasonic observation apparatus, the image processing unit is configured to generate feature image data based on the feature. When the one of the features is re-extracted, the image processing unit is configured to generate feature image data based on the one of the features re-extracted by use of the parameters used for generating feature image data based on the other of the features.

In the above-mentioned ultrasonic observation apparatus, the image processing unit is configured to further generate differential image data indicating a difference between first feature image data generated based on the one of the features re-extracted and second feature image data generated based on the other of the features.

In the above-mentioned ultrasonic observation apparatus, the image processing unit is configured to generate B-mode image data based on the received ultrasonic wave. When the differential image data is generated, the image processing unit is configured to further generate combined image data in which the differential image data and the B-mode image data are combined.

In the above-mentioned ultrasonic observation apparatus, the image processing unit is configured to further generate display image data indicating a screen including: at least one of a B-mode image based on the B-mode image data and a feature image based on the feature image data; and a combined image based on the combined image data.

In the above-mentioned ultrasonic observation apparatus, when the one of the features is re-extracted, the image processing unit is configured to further generate image data indicating a graph or table of a comparison between the one of the features re-extracted and the other of the features.

In the above-mentioned ultrasonic observation apparatus, the position information acquisition unit includes: a probe position information acquisition unit configured to acquire position information indicating a position of the ultrasound probe; a patient position information acquisition unit configured to acquire position information indicating a position of the patient; and a position information calculation unit configured to calculate a relative position coordinate of the ultrasound probe relative to the patient, based on the position information of the ultrasound probe and the position information of the patient.

In the above-mentioned ultrasonic observation apparatus, the probe position information acquisition unit includes: a first marker provided on the ultrasound probe; and a sensor unit configured to detect the first marker and to output a detection signal. The patient position information acquisition unit includes: a second marker configured to be mounted on a body surface of the patient; and an optical camera configured to image the second marker to generate an image.

In the above-mentioned ultrasonic observation apparatus, the computation unit is configured to perform a frequency spectrum analysis on the received ultrasonic wave to calculate a frequency spectrum, and to extract the feature by use of a result of an approximation processing for the frequency spectrum.

In the above-mentioned ultrasonic observation apparatus, the computation unit is configured to extract a harmonic signal from the received ultrasonic wave, and to extract an amplitude of an envelope of the harmonic signal as the feature.

In the above-mentioned ultrasonic observation apparatus, the computation unit is configured to measure a distortion amount in the specimen based on the received ultrasonic wave, and to extract the distortion amount as the feature.

A method according to the invention is a method for operating an ultrasonic observation apparatus for transmitting an ultrasonic wave toward a specimen and receiving the ultrasonic wave reflected from the specimen by an ultrasound probe to generate an image based on the received ultrasonic wave. The method includes: a computation step of extracting, by a computation unit, a feature of the received ultrasonic wave; an image processing step of generating, by an image processing unit, image data based on the received ultrasonic wave; a position information acquisition step of acquiring, by a position information acquisition unit, relative position information indicating a relative position of the ultrasound probe relative to a patient as an examination target; a data selection step of searching, by a data selection unit, a plurality of data sets stored in a storage unit based on the relative position information acquired in the position information acquisition step, and selecting a data set meeting a predetermined condition, the storage unit storing the plurality of data sets including: position information on an observation point; the feature extracted by the computation unit for the observation point; the image data generated by the image processing unit for the observation point; and parameters used for extracting the feature and generating the image data such that the plurality of data sets associates with information for identifying the patient; and an execution control step of, when the data set meeting the predetermined condition is selected, causing, by an execution control unit, the computation unit to re-extract one of a feature included in the data set selected by the data selection unit and a latest feature extracted by the computation unit by use of the parameters used for extracting the other of the features.

A program according to the invention is a program for operating an ultrasonic observation apparatus for transmitting an ultrasonic wave toward a specimen and receiving the ultrasonic wave reflected from the specimen by an ultrasound probe to generate an image based on the received ultrasonic wave. The program causes the ultrasonic observation apparatus to execute: a computation step of extracting, by a computation unit, a feature of the received ultrasonic wave; an image processing step of generating, by an image processing unit, image data based on the received ultrasonic wave; a position information acquisition step of acquiring, by a position information acquisition unit, relative position information indicating a relative position of the ultrasound probe relative to a patient as an examination target; a data selection step of searching, by a data selection unit, a plurality of data sets stored in a storage unit based on the relative position information acquired in the position information acquisition step, and selecting a data set meeting a predetermined condition, the storage unit storing the plurality of data sets including: position information on an observation point; the feature extracted by the computation unit for the observation point; the image data generated by the image processing unit for the observation point; and parameters used for extracting the feature and generating the image data such that the plurality of data sets associates with information for identifying the patient; and an execution control step of, when the data set meeting the predetermined condition is selected, causing, by an execution control unit, the computation unit to re-extract one of a feature included in the data set selected by the data selection unit and a latest feature extracted by the computation unit by use of the parameters used for extracting the other of the features. Advantageous Effects of Invention

According to the present invention, when a plurality of data sets stored in the storage unit are searched based on the relative position information of the ultrasound probe relative to a patient and a data set meeting the predetermined condition is selected, one of the latest feature extracted by the computation unit and the feature included in the selected data set is re-extracted by use of the parameters used for extracting the other of the features, and thus both of the features can be compared with each other and a user can directly grasp a temporal change in a site under observation.

Brief Description of Drawings

FIG. 1 is a block diagram illustrating an exemplary structure of an ultrasonic observation apparatus according to a first embodiment of the present invention.
FIG. 2 is a schematic diagram illustrating an exemplary structure of the ultrasonic observation apparatus according to the first embodiment of the present invention.
FIG. 3 is a schematic diagram for explaining a data structure of examination data stored in an examination data storage unit illustrated in FIG. 1.
FIG. 4 is a flowchart illustrating the operations of the ultrasonic observation apparatus illustrated in FIG. 1.
FIG. 5 is a schematic diagram illustrating a state in which an insertion part of an ultrasonic endoscope is inserted into the body of a patient.
FIG. 6 is a schematic diagram illustrating exemplary display of a B-mode image.
FIG. 7 is a flowchart illustrating a frequency analysis processing performed by a feature analysis unit illustrated in FIG. 1.
FIG. 8 is a diagram schematically illustrating a data arrangement of an acoustic ray.
FIG. 9 is a diagram illustrating an exemplary frequency spectrum calculated by the feature analysis unit illustrated in FIG. 1.
FIG. 10 is a schematic diagram for explaining a record data determination method performed by a data selection unit illustrated in FIG. 1.
FIG. 11 is a schematic diagram for explaining the record data determination method performed by the data selection unit illustrated in FIG. 1.

FIG. 12 is a schematic diagram illustrating exemplary display of a display screen including a frequency feature image and the frequency feature.

FIG. 13 is a schematic diagram illustrating an exemplary screen displayed when determining that past record data meeting a condition is present.

FIG. 14 is a schematic diagram illustrating exemplary display of a display screen including a B-mode image and a combined image in which a differential image is overlapped on the B-mode image.

FIG. 15 is a block diagram illustrating an exemplary structure of an ultrasonic observation apparatus according to a third modification of the first embodiment of the present invention.

FIG. 16 is a schematic diagram illustrating another exemplary display of the display screen including a B-mode image and a combined image in which a differential image is overlapped on the B-mode image.

FIG. 17 is a schematic diagram for explaining an attenuation correction method according to a seventh modification of the first embodiment of the present invention.

Description of Embodiments

Embodiments of an ultrasonic observation apparatus, a method for operating the ultrasonic observation apparatus, and a program for operating the ultrasonic observation apparatus according to the present invention will be described below in detail with reference to the drawings. The present invention is not limited to the embodiments. The same reference signs are used to designate the same elements throughout the drawings.

(First Embodiment)

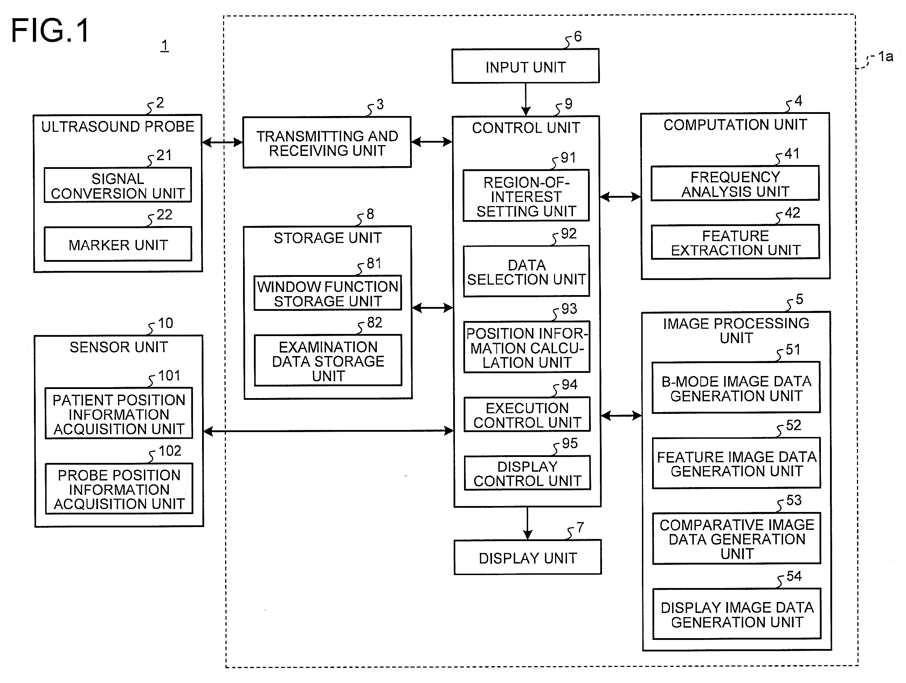

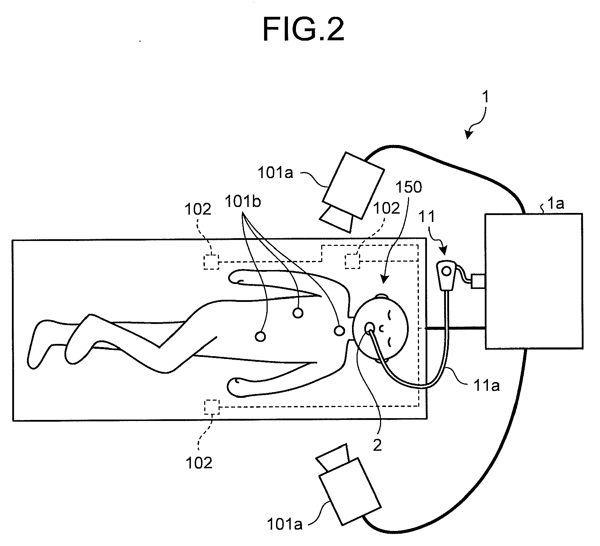

FIG. 1 is a block diagram illustrating an exemplary structure of an ultrasonic observation apparatus according to a first embodiment of the present invention. The illustrated ultrasonic observation apparatus 1 is an apparatus for observing a specimen by use of ultrasonic waves. FIG. 2 is a schematic diagram illustrating an exemplary structure of the ultrasonic observation apparatus 1.

The ultrasonic observation apparatus 1 includes an ultrasound probe 2 for outputting an ultrasonic pulse to the outside and receiving an ultrasonic echo reflected in the outside, a transmitting and receiving unit 3 for transmitting and receiving an electric signal to/from the ultrasound probe 2, a computation unit 4 for performing a predetermined computation processing on an electric echo signal as a converted ultrasonic echo, an image processing unit 5 for generating image data corresponding to an electric echo signal as a converted ultrasonic echo, an input unit 6 for inputting therein various items of information on the ultrasonic observation apparatus 1, a display unit 7 realized by use of a liquid crystal or organic EL display panel and directed for displaying various items of information including an image generated by the image processing unit 5, a storage unit 8 for storing various items of information such as parameters used for the computation processing and the image processing on an echo signal or the results of the processings, a control unit 9 for controlling the operations of the ultrasonic observation apparatus 1, and a sensor unit 10 as a position information acquisition unit for acquiring relative position information indicating a relative position relationship of the ultrasound probe 2 relative to a patient 150.

The ultrasound probe 2 includes a signal conversion unit 21 formed of a plurality of ultrasonic transducers for converting and transmitting an electric pulse signal received from the transmitting and receiving unit 3 into an ultrasonic pulse (acoustic pulse signal) and receiving and converting an ultrasonic echo reflected from a specimen into an electric echo signal, and a marker unit 22 for sensing a position of the ultrasound probe 2. The ultrasound probe 2 may be directed for transmitting ultrasonic waves in a predetermined direction and scanning a specimen under mechanical control on the ultrasonic transducers, or may be directed for transmitting ultrasonic waves in a predetermined direction and scanning a specimen under electronic control on the ultrasonic transducers (also referred to as electronic scan).

As illustrated in FIG. 2, according to the first embodiment, there will be described an example in which the present invention is applied to an ultrasonic endoscope 11 which is provided with the ultrasound probe 2 at the tip end of an insertion part 11a to be inserted into the body of the patient 150 and is directed for observing the inside of the body of the patient 150 by ultrasonic waves. Of course, the present invention is applicable to typical external ultrasound probes.

The marker unit 22 is made of a member detectable by the sensor unit 10 described later. As in the first embodiment, when the ultrasound probe 2 is inserted into the body of the patient 150, the marker unit 22 may be made of a magnetic field generation member detectable from the outside of the patient 150, such as permanent magnet or coil for generating a magnetic field due to a current flow, by way of example.

The transmitting and receiving unit 3 is electrically connected to the ultrasound probe 2, and is directed to transmit a pulse signal to the ultrasound probe 2 and to receive an echo signal from the ultrasound probe 2. More specifically, the transmitting and receiving unit 3 generates a pulse signal based on a preset waveform and a transmission timing and transmits the generated pulse signal to the ultrasound probe 2. Further, the transmitting and receiving unit 3 performs the processings such as amplification and filtering on the received echo signal to be subjected to A/D conversion, and generates and outputs a digital RF signal. When the ultrasound probe 2 is of an electronic scan type, the transmitting and receiving unit 3 has a multi-channel circuit for beam combination corresponding to the ultrasonic transducers. In the following, the digital RF signal generated by the transmitting and receiving unit 3 will be referred to as acoustic ray data.

The computation unit 4 extracts the feature from the acoustic ray data output from the transmitting and receiving unit 3. In other words, it acquires the feature of the specimen in an ultrasonic echo reception direction. In the first embodiment, the frequency feature is extracted as an example of the feature. More specifically, the computation unit 4 according to the first embodiment includes a frequency analysis unit 41 for performing fast Fourier transformation (FFT) on the acoustic ray data output from the transmitting and receiving unit 3 and performing the frequency analysis to calculate a frequency spectrum, and a feature extraction unit 42 for performing an approximation processing based on regression analysis and an attenuation correction processing of reducing a contribution of attenuation caused depending on a reception depth and a frequency of an ultrasonic wave when the ultrasonic wave propagates to extract the feature of the specimen.

The frequency analysis unit 41 calculates frequency spectra at a plurality of portions (data positions) on an acoustic ray by performing fast Fourier transformation on FFT data groups made of a predetermined amount of data for each line of acoustic ray data. Generally, the frequency spectrum indicates a different trend depending on tissue characteristics of the specimen. This is because the frequency spectrum has a correlation with a size, a density, an acoustic impedance, and the like of the specimen as a scattering body for scattering ultrasonic waves. In the first embodiment, examples of "tissue characteristics" include any of cancer, endocrine tumor, mucinous tumor, normal tissue, and vascular channel, for example.

The feature extraction unit 42 approximates a frequency spectrum by a primary expression (regression line) based on regression analysis, thereby extracting the feature before attenuation correction characterizing the approximated primary expression (which will be called pre-correction feature). Specifically, the feature extraction unit 42 first extracts a slope $a_0$ and an intercept $b_0$ of the primary expression as the pre-correction features. An approximation unit 124 may calculate an intensity (which is also called Mid-band fit) of $c_0 = a_0 f_M + b_0$ at a center frequency of $f_M = (f_L + f_H)/2$ of a frequency band ($f_L < f < f_H$) as the pre-correction feature other than the slope $a_0$ and the intercept $b_0$.

The slope $a_0$ among the three features is considered as having a correlation with a size of the ultrasonic scattering body and generally having a smaller value of the slope as the scattering body is larger. The intercept $b_0$ has a correlation with a size of the scattering body, a difference in acoustic impedance, a density (concentration) of the scattering body, and the like. Specifically, the intercept $b_0$ is considered as having a larger value as the scattering body is larger, having a larger value as the acoustic impedance is larger, and having a larger value as the density (concentration) of the scattering body is larger. The intensity $c_0$ at the center frequency $f_M$ (which will be simply called "intensity" below) is an indirect parameter derived from the slope $a_0$ and the intercept $b_0$, and gives a spectrum intensity at the center of an effective frequency band. Therefore, the intensity $c_0$ is considered as having a certain correlation with a luminance of a B-mode image in addition to the size of the scattering body, the difference in acoustic impedance, and the density of the scattering body. An approximate polynomial calculated by the feature extraction unit 42 is not limited to the primary expression, and approximate polynomials of degree of two or more may be employed.

Subsequently, the feature extraction unit 42 performs an attenuation correction processing of reducing a contribution of attenuation caused depending on a reception depth and a frequency of an ultrasonic wave when the ultrasonic wave propagates. Generally, the ultrasonic attenuation amount A(f, z) is expressed as:

$$A(f, z) = 2\alpha z f \cdots (1)$$

Herein, $\alpha$ denotes an attenuation rate, z denotes an ultrasonic reception depth, and f denotes a frequency. As is clear from Equation (1), the attenuation amount A(f, z) is proportional to the frequency f. When an object to be observed is a living body, a specific value of the attenuation rate $\alpha$ is 0.0 to 1.0 (dB/cm/MHz), more preferably 0.3 to 0.7 (dB/cm/MHz), that is, defined depending on a site of the living body. For example, when an object to be observed is the spleen, $\alpha = 0.6$ (dB/cm/MHz) may be defined. According to the first embodiment, a value of the attenuation rate $\alpha$ may be set or changed by input of the input unit 6.

The feature extraction unit 42 extracts the feature by performing the attenuation correction on the pre-correction feature (slope $a_0$, intercept $b_0$, intensity $c_0$) acquired by the approximation processing as follows.

$$a = a_0 + 2\alpha z \cdots (2)$$

$$b = b_0 \cdots (3)$$

$$c = c_0 + 2\alpha z f_M \ (= a f_M + b) \cdots (4)$$

As is clear from Equations (2) and (4), the feature extraction unit 42 performs the correction such that the correction amount is larger as the ultrasonic reception depth z becomes larger. Further, from Equation (3), the correction of the intercept is identical transformation. This is because the intercept is a frequency component corresponding to the frequency 0(Hz) and is not influenced by the attenuation.

A line corresponding to the corrected feature is expressed in the following Equation.

$$I = a f + b = (a_0 + 2\alpha z) f + b_0 \cdots (5)$$

As is clear from Equation (5), the line corresponding to the corrected feature is larger in the slope than and has the same intercept as the line corresponding to the pre-correction feature.

More preferably, the computation unit 4 is provided with an amplification correction unit for performing amplification correction on the acoustic ray data output by the transmitting and receiving unit 3 such that an amplification rate is constant irrespective of the reception depth. Herein, generally, STC (Sensitivity Time Control) correction is made to uniformly amplify an amplitude of an analog signal waveform over the entire frequency band in the transmitting and receiving unit 3. When a B-mode image using an ultrasonic amplitude is generated, a sufficient effect can be obtained by the STC correction, while when an ultrasonic frequency spectrum is calculated, an influence caused by the attenuation due to propagation of the ultrasonic wave cannot be accurately removed. In order to solve the problem, there is assumed that while a reception signal subjected to the STC correction is output for generating a B-mode image, when an image based on a frequency spectrum is generated, new transmission different from the transmission for generating the B-mode image is performed to output a reception signal not subjected to the STC correction. In this case, however, there is a problem that a frame rata of image data generated based on the reception signal lowers. Thus, the amplification rate is corrected on the signal subjected to the STC correction for the B-mode image in front of the frequency analysis unit 41 in order to once remove an influence of the STC correction while keeping the frame rate of the generated image data.

The image processing unit 5 includes a B-mode image data generation unit 51 for generating B-mode image data directed for converting an amplitude of acoustic ray data into luminance for display, a feature image data generation unit 52 for generating feature image data directed for converting the feature extracted from acoustic ray data into luminance for display, a comparative image data generation unit 53 for generating differential image data indicating a difference from the image data stored for a past examination of the same patient, and a display image data generation unit 54 for creating image data for display by use of the image data generated in each unit.

The B-mode image data generation unit 51 generates B-mode image data by performing a signal processing using a well-known technique such as bandpass filter, logarithmic conversion, gain processing and contrast processing on a digital RF signal (acoustic ray data) and by decimating data depending on a data step width defined depending on an image display range in the display unit 7.

According to the first embodiment, the feature image data generation unit 52 converts the frequency feature extracted by the feature extraction unit 42 into a pixel value to generate feature image data. Information allocated to each pixel in the feature image data is defined depending on the data amount in an FFT data group when the frequency analysis unit 41 calculates a frequency spectrum. Specifically, information corresponding to the feature of a frequency spectrum calculated from one FFT data group is allocated to a pixel region corresponding to the data amount of the FFT data group, for example. According to the first embodiment, the number of features used for generating the feature image data can be arbitrarily set.

The comparative image data generation unit 53 calculates a difference between the feature image data based on the feature extracted at a real-time (latest) observation point and the feature image data included in the data selected by a data selection unit 92 (that is, the feature image data at a past observation point identical or close to the latest observation point) to generate differential image data.

The display image data generation unit 54 generates the image data indicating a graph or table for comparing the feature extracted at a real-time (latest) observation point with the feature included in the data selected by the data selection unit 92, or combined image data using the B-mode image data and the differential image data, and creates image data for displaying the screen based on the image data on the display unit 7.

The input unit 6 is realized by use of an interface such as keyboard, mouse, touch panel, or card reader, and inputs a signal depending on an operation externally performed by the operator or the like into the control unit 9. Specifically, the input unit 6 receives patient identification information for specifying the patient 150, a region-of-interest setting instruction, instructions to start various operations, and the like, and inputs the signals indicating the information or instructions into the control unit 9. Herein, the region of interest is a region in an image designated by the operator of the ultrasonic observation apparatus 1 via the input unit 6 for the B-mode image displayed on the display unit 7.

The storage unit 8 is realized by a ROM for previously storing therein a program for operating the ultrasonic observation apparatus 1 according to the first embodiment, a program for activating the predetermined OS, and the like, a RAM for storing parameters, data, and the like used for each processing, or the like. More specifically, the storage unit 8 includes a window function storage unit 81 for storing therein window functions used for the frequency analysis processing performed by the frequency analysis unit 41, and an examination data storage unit 82 for storing therein examination data including frequency analysis results per observation point where the observation is made.

The window function storage unit 81 stores at least one window function among the window functions such as Hamming, Hanning, and Blackman.

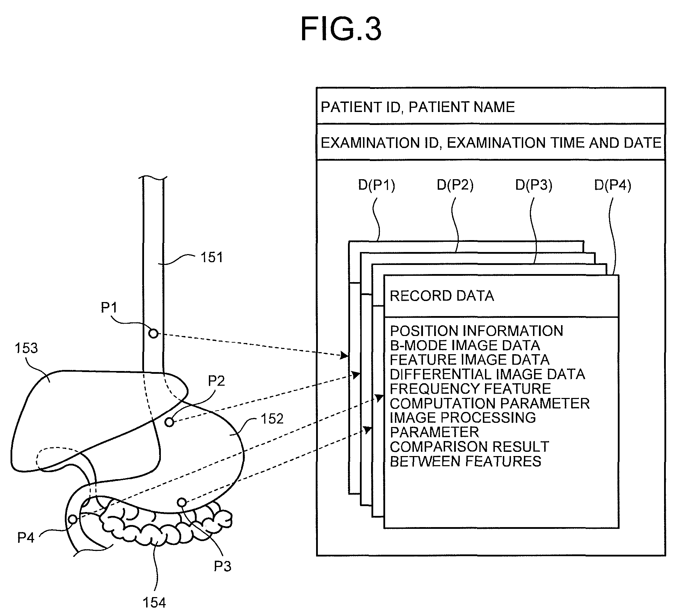

FIG. 3 is a schematic diagram for explaining a data structure of examination data stored in the examination data storage unit 82. In FIG. 3, organs in the patient 150 (esophagus 151, stomach 152, liver 153, spleen 154) are displayed in association with the record data at the observation points P1 to P4 where the ultrasonic observation is made in the organs.

As illustrated in FIG. 3, the examination data storage unit 82 stores patient identification information (such as patient ID and patient name) for specifying the patient 150, examination identification information (such as examination ID and examination time and date) for specifying an examination, and record data D(P1) to D(P4) created per observation point P1 to P4 for each examination made on the patient 150. Each of the record data D(P1) to D(P4) is a data set including position information on the observation points P1 to P4, image data generated by the image processing unit 5 for the observation points P1 to P4, the feature (such as the frequency feature) acquired by the computation unit for the observation points P1 to P4, computation parameters used for acquiring the feature, image processing parameters used for generating the image data, comparison results with the features of past examinations, and the like. The image data among them includes B-mode image data, feature image data, or differential image data. Further, the computation parameters include a size and position of a region of interest for extracting the feature, an attenuation correction coefficient, a frequency spectrum approximation method, a window function, and the like. The image processing parameters include gain for generating the B-mode image and the feature image, contrast, $\gamma$-correction coefficient, and the like.

The control unit 9 includes a region-of-interest setting unit 91 for setting a region of interest for the B-mode image according to a region-of-interest setting instruction input from the input unit 6, the data selection unit 92 for acquiring information meeting a predetermined condition from the storage unit 8 based on the relative position information of the ultrasound probe 2 relative to the patient 150, a position information calculation unit 93 for calculating a relative position coordinate of the ultrasound probe 2 relative to the patient 150 based on the information output from the sensor unit 10, an execution control unit 94 for controlling to execute the computation processing in the computation unit 4 and the image processing in the image processing unit 5, and a display control unit 95 for controlling a display operation in the display unit 7.

The data selection unit 92 searches the examination data stored in the storage unit 8 based on the relative position coordinate of the ultrasound probe 2 calculated by the position information calculation unit 93 thereby to select data on an observation point identical or close to the latest observation point acquired for the examination past made on the same patient as the patient 150 to be examined.

The position information calculation unit 93 calculates a position coordinate of the patient 150 based on information output from a patient position information acquisition unit 101 and stores it as reference position information in the storage unit 8, calculates a position coordinate of the ultrasound probe 2 based on information output from a probe position information acquisition unit 102, and converts the position coordinate of the ultrasound probe 2 into a relative position coordinate relative to the patient 150 based on the reference position information. Then, the relative position coordinate is stored as the position information on the observation point in the storage unit 8 in association with the data on the site under observation.

The sensor unit 10 includes the patient position information acquisition unit 101 for acquiring a position or posture of the patient 150, and the probe position information acquisition unit 102 for acquiring a position or posture of the ultrasound probe 2.

As illustrated in FIG. 2, the patient position information acquisition unit 101 includes, for example, two optical cameras 101a and reference markers 101b mounted on the body surface of the patient 150. The reference marker 101b employs an object easily detectable in an image captured by the optical camera 101a, such as conspicuously-colored ball or disk. The reference markers 101b are arranged on at least three positions on the body surface of the patient 150. The two optical cameras 101a are arranged where the reference markers 101b are within each field of view and the reference markers 101b can be captured in mutually different directions.

Each optical camera 101a outputs image data generated by capturing the reference markers 101b. Accordingly, the position information calculation unit 93 detects the positions of the reference markers 101b from each of the two images capturing the reference markers 101b therein, and measures the position of each reference marker 101b with a well-known stereovision method. The thus-acquired position information on at least three reference markers 101b is stored as position information (reference position information) of the patient 150 for the examination in the storage unit 8.

The structure of the patient position information acquisition unit 101 is not limited to the structure including the optical cameras 101a and the reference markers 101b. For example, the patient position information acquisition unit 101 may include at least three reference markers made of magnet and a plurality of magnetic sensors for detecting magnetic fields generated from the reference markers at mutually different positions, for example.

The probe position information acquisition unit 102 is configured depending on the marker unit 22 provided on the ultrasound probe 2. For example, if the marker unit 22 is formed of a permanent magnet or coil, the probe position information acquisition unit 102 includes a plurality of magnetic sensors. In this case, the probe position information acquisition unit 102 detects a magnetic field generated from the marker unit 22 and outputs a detection signal indicating an intensity of the magnetic field. Accordingly the position information calculation unit 93 calculates a position coordinate of the marker unit 22. Further, the position information calculation unit 93 converts the position coordinate of the marker unit 22 into a relative position coordinate based on the reference position information, and outputs it as relative position information on the ultrasound probe 2 at that point of time.

The sensor unit 10 and the position information calculation unit 93 constitutes a relative position information acquisition unit for acquiring relative position information indicating a relative position of the ultrasound probe 2 relative to the patient 150.

The components other than the ultrasound probe 2 and the sensor unit 10 in the ultrasonic observation apparatus 1 having the above functional structure are realized by use of a computer 1a including the CPU with the computation and control functions. The CPU provided in the ultrasonic observation apparatus 1 reads the information and various programs including the program for operating the ultrasonic observation apparatus 1 stored and saved in the storage unit 8 from the storage unit 8 to perform the computation processing associated with the method for operating the ultrasonic observation apparatus 1 according to the first embodiment.

The program for operating the ultrasonic observation apparatus according to the first embodiment may be recorded in a computer readable recording medium such as hard disk, flash memory, CD-ROM, DVD-ROM, or flexible disk to be widely distributed.

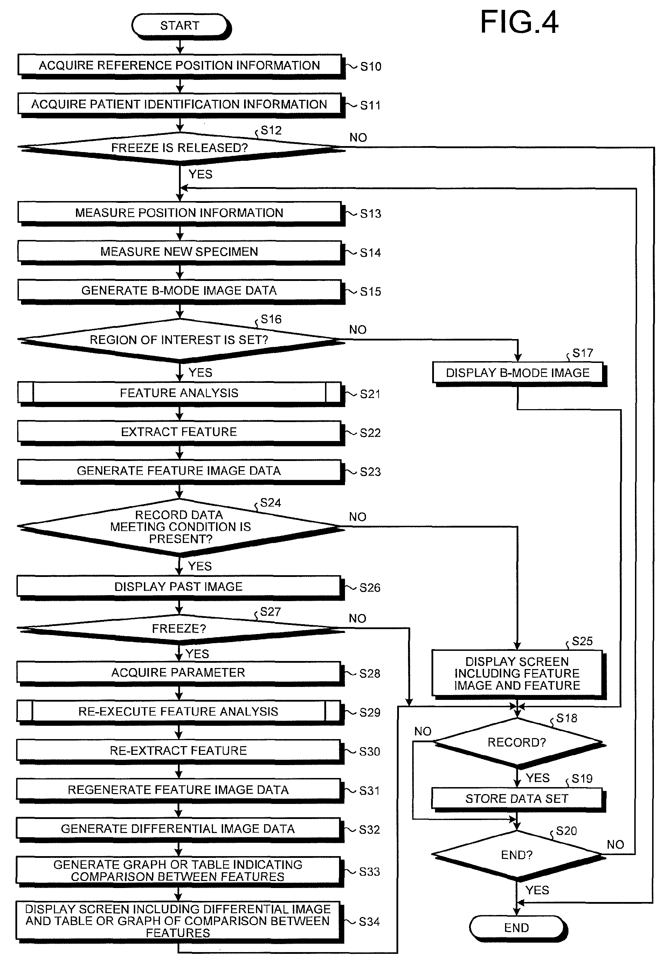

The operations of the ultrasonic observation apparatus 1 will be described below. FIG. 4 is a flowchart illustrating the operations of the ultrasonic observation apparatus 1.

At first, in step S10, the ultrasonic observation apparatus 1 acquires the reference position information indicating a position of the patient 150. That is, as illustrated in FIG. 2, at least three reference markers 101b are captured by the two optical cameras 101a, and the positions of the reference markers 101b are measured based on the thus-acquired images to assume the measurement result as the reference position information. At least three reference markers 101b are employed so that a plan passing through the predetermined positions on the body surface of the patient 150 can be set as a reference surface.

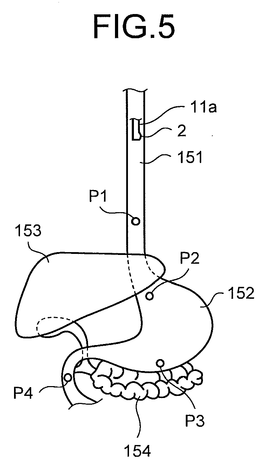

In subsequent step S11, the ultrasonic observation apparatus 1 acquires the patient identification information including patient ID, patient name, date of birth, sex, and the like. The ultrasonic observation apparatus 1 acquires the patient identification information according to the input operations performed on the input unit 6. Specifically, the patient identification information can be acquired according to text input via the keyboard or predetermined mouse operations. Alternatively, the patient identification information may be acquired by reading a barcode described on the medical record of the patient by a barcode reader. Further, the patient identification information may be acquired via a network server. Thereafter, as illustrated in FIG. 5, the insertion part 11a of the ultrasonic endoscope 11 is inserted into the patient 150.

In step S12, when a freeze releasing instruction signal is input from the input unit 6 (step S12: Yes), the ultrasonic observation apparatus 1 starts measuring the position information of the ultrasound probe 2 (step S13). That is, the probe position information acquisition unit 102 starts operating under control of the control unit 9, and accordingly the position information calculation unit 93 acquires a detection signal output from the probe position information acquisition unit 102 to calculate a position coordinate of the marker unit 22, and calculates the relative position information of the ultrasound probe 2 relative to the patient 150 based on the reference position information.

On the other hand, when a freeze releasing instruction signal is not input (step S12: No), the ultrasonic observation apparatus 1 terminates the operation.

In step S14 subsequent to step S13, the ultrasonic observation apparatus 1 measures a novel specimen by the ultrasound probe 2. That is, an ultrasonic pulse is transmitted from the ultrasound probe 2 toward the specimen, and an ultrasonic echo reflected from the specimen is received and the ultrasonic echo is converted into an electric signal, and then into a digital signal to acquire acoustic ray data.

In subsequent step S15, the B-mode image data generation unit 51 generates B-mode image data based on the acoustic ray data acquired in step S14. The B-mode image is a gray scale image in which the values of R(red), G(green), and B(blue), as variables when the RGB display color system is employed as a color space, are matched.

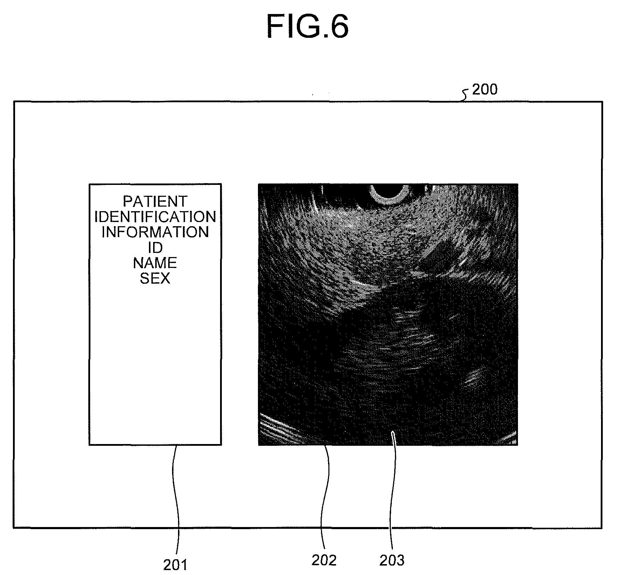

At this time, when a region of interest is not set (step S16: No), the control unit 9 controls to display the B-mode image on the display unit 7 based on the B-mode image data generated by the B-mode image data generation unit 51 (step S17). FIG. 6 is a schematic diagram illustrating exemplary display of the B-mode image. A display screen 200 illustrated in FIG. 6 includes an information display region 201 for the patient identification information such as ID, name, and sex, and an image display region 202. The image display region 202 displays therein a B-mode image 203 based on the ultrasonic echo received by the ultrasound probe 2.

Thereafter, when a data recording instruction signal is input from the input unit 6 (step S18: Yes), the control unit 9 stores the relative position information of the ultrasound probe 2 at this time as position information on the observation point in the examination data storage unit 82 as one data set together with the B-mode image data and the image processing parameters used for generating the B-mode image (step S19). Thereafter, the operation of the ultrasonic observation apparatus 1 proceeds to step S20.

On the other hand, when a data recording instruction signal is not input (step S18: No), the operation of the ultrasonic observation apparatus 1 proceeds to step S20.

In step S20, when an operation terminating instruction is input by the input unit 6 (step S20: Yes), the ultrasonic observation apparatus 1 terminates the operation. To the contrary, when an operation terminating instruction is not input by the input unit 6 (step S20: No), the operation of the ultrasonic observation apparatus 1 returns to step S13.

On the other hand, in step S16, when a region of interest is set via the input unit 6 (step S16: Yes), the computation unit 4 performs the feature analysis on the acoustic ray data acquired in step S14 (step S21). According to the first embodiment, as one example of the feature analysis, the frequency analysis unit 41 performs the frequency analysis by the FFT computation to calculate a frequency spectrum. In the frequency analysis, the entire region of the image may be set as a region of interest.

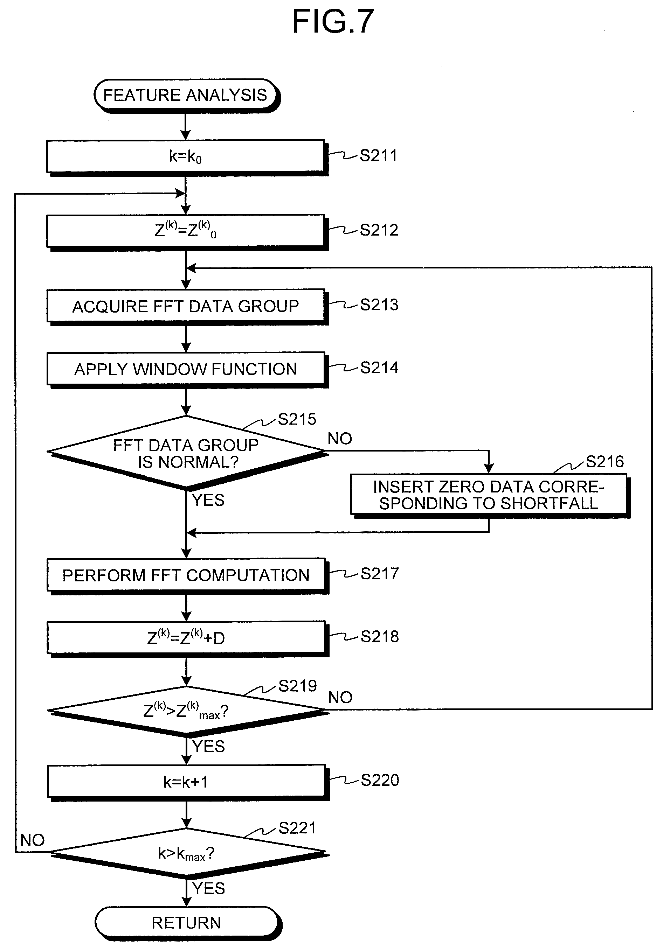

FIG. 7 is a flowchart illustrating the feature analysis processing (frequency analysis processing).

At first, the frequency analysis unit 41 sets a counter k for identifying an acoustic ray to be analyzed as $k_0$ (step S211).

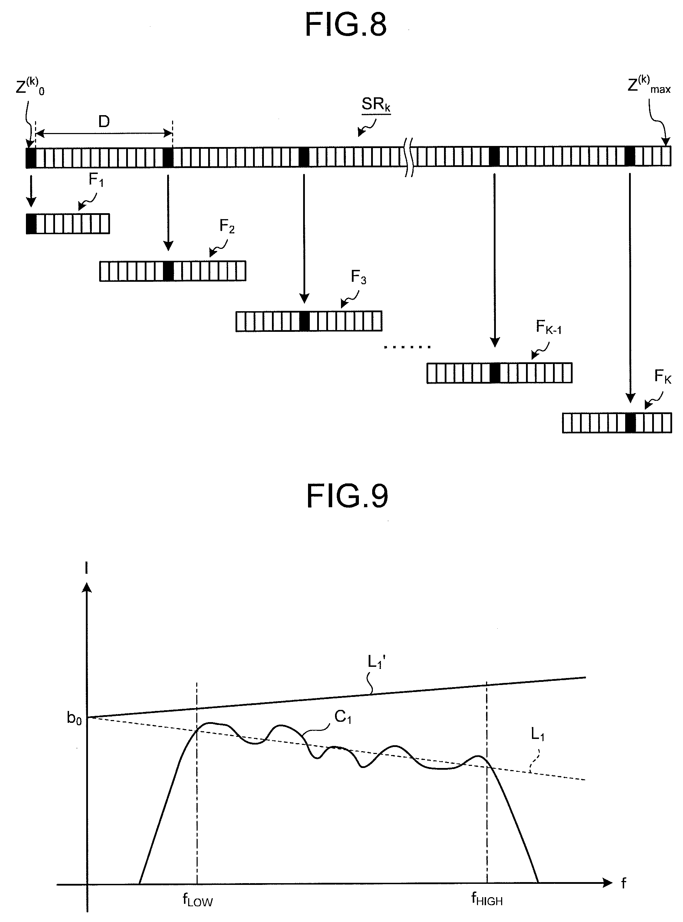

Subsequently, the frequency analysis unit 41 sets an initial value $Z^{(k)}_0$ of a representative data position (corresponding to reception depth) $Z^{(k)}$ of a series of data groups (FFT data groups) acquired for the FFT computation (step S212). FIG. 8 is a diagram schematically illustrating a data arrangement of one acoustic ray. For the illustrated acoustic ray $SR_k$, a white or black rectangle indicates one item of data. The acoustic ray $SR_k$ is discrete at time intervals corresponding to a sampling frequency (such as 50 MHz) for A/D conversion performed by the transmitting and receiving unit 3. FIG. 8 illustrates a case in which the first data position of the acoustic ray $SR_k$ is set at the initial value $Z^{(k)}_0$, but the position of the initial value may be arbitrarily set.

Thereafter, the frequency analysis unit 41 acquires an FFT data group of the data position $Z^{(k)}$ (step S213), and applies the window function stored in the window function storage unit 81 to the acquired FFT data group (step S214). The window function is operated on the FFT data group in this way, thereby avoiding a discontinuous border of the FFT data group and preventing artifacts from occurring.

Subsequently, the frequency analysis unit 41 determines whether the FFT data group of the data position $Z^{(k)}$ is a normal data group (step S215). Herein, the FFT data group needs to have a data number with a power of 2. In the following, the data number of an FFT data group is assumed as $2^n$ (n is a positive integer). The fact that an FFT data group is normal indicates that the data position $Z^{(k)}$ is the $2^{n-1}$-th position from the head of the FFT data group. In other words, the fact that an FFT data group is normal indicates that $2^{n-1}-1$ (= N) items of data are present ahead of the data position $Z^{(k)}$ and $2^{n-1}$ (= M) items of data are present behind the data position $Z^{(k)}$. In the case illustrated in FIG. 8, the FFT data groups $F_2$ and $F_3$ are both normal. FIG. 8 illustrates the case with n = 4 (N = 7, M = 8) by way of example.

As a determination result in step S215, when the FFT data group of the data position $Z^{(k)}$ is normal (step S215: Yes), the frequency analysis unit 41 proceeds to step S217 described below.

As a determination result in step S215, when the FFT data group of the data position $Z^{(k)}$ is not normal (step S215: No), the frequency analysis unit 41 inserts zero data corresponding to the shortfall to generate a normal FFT data group (step S216). The FFT data group determined as not normal in step S215 is subjected to the window function before the zero data is added. Therefore, even if the zero data is inserted into the FFT data group, discontinuous data does not occur. After step S216, the frequency analysis unit 41 proceeds to step S217 described below.

In step S217, the frequency analysis unit 41 performs FFT computation using the FFT data group to acquire a frequency spectrum made of a complex number (step S217). Consequently, a spectrum $C_1$ as illustrated in FIG. 9 is acquired, for example.

Subsequently, the frequency analysis unit 41 changes the data position $Z^{(k)}$ by a step width D (step S218). The step width D is assumed to be previously stored in the storage unit 8. FIG. 8 illustrates the case with D = 15 by way of example. It is desirable that the step width D matches with a data step width used by the B-mode image data generation unit 51 for generating B-mode image data, but when the computation amount in the frequency analysis unit 41 is desired to reduce, the data step width D may be set at a larger value than the data step width.

Thereafter, the frequency analysis unit 41 determines whether the data position $Z^{(k)}$ is larger than the maximum

value $Z^{(k)}_{max}$ in the acoustic ray $SR_k$ (step S219). When the data position $Z^{(k)}$ is larger than the maximum value $Z^{(k)}_{max}$ (step S219: Yes), the frequency analysis unit 41 increments the counter k by 1 (step S220). On the other hand, when the data position $Z^{(k)}$ is equal to or smaller than the maximum value $Z^{(k)}_{max}$ (step S219: No), the frequency analysis unit 41 returns to step S213. In this way, the frequency analysis unit 41 performs the FFT computation on the $[\{(Z^{(k)}_{max} - Z^{(k)}_0)/D\} + 1]$ FFT data groups in the acoustic ray $SR_k$. Herein, [X] denotes a maximum integer not exceeding X.

After step S220, the frequency analysis unit 41 determines whether the counter k is larger than the maximum value $k_{max}$ (step S221). When the counter k is larger than the maximum value $k_{max}$ (step S221: Yes), the frequency analysis unit 41 terminates a series of FFT computations. On the other hand, when the counter k is equal to or smaller than $k_{max}$ (step S221: No), the frequency analysis unit 41 returns to step S212.

In this way, the frequency analysis unit 41 performs the FFT computation several times on each of ($k_{max} - k_0$ + 1) acoustic rays.

Herein, the frequency analysis processing is performed only within the region of interest in response to the previously-input setting of the specific region of interest by the input unit 6, but the frequency analysis processing may be performed on all the regions in which the frequency analysis unit 41 receives an ultrasonic signal.

In step S22 subsequent to step S21, the computation unit 4 extracts the feature from the acoustic ray data based on the result of the feature analysis. According to the first embodiment, the feature extraction unit 42 performs the regression analysis on P frequency spectra calculated by the frequency analysis unit 41 and further performs the attenuation correction to extract the feature. Specifically, the feature extraction unit 42 calculates a primary expression for approximating a frequency spectrum with a frequency band of $f_{LOW} < f < f_{HIGH}$ by the regression analysis to calculate three pre-correction features $a_0$, $b_0$, and $c_0$. The line $L_1$ indicated in FIG. 9 is a pre-correction regression line acquired in the processing.

The feature extraction unit 42 further substitutes the value of the data position $Z^{(k)}$ into the reception depth z in Equations (2) to (4) to calculate the slope a, the intercept b, and the intensity c as the corrected features. The line $L_1$' indicated in FIG. 9 is a regression line acquired in step S22.

In subsequent step S23, the image processing unit 5 generates feature image data based on the feature extracted in step S22. According to the first embodiment, the feature image data generation unit 52 generates the feature image data based on the frequency feature extracted in step S22. Specifically, the feature image data is gray scale image data in which the intercept b is uniformly allocated to R(red), G(green), and B(blue) of each pixel in the region of interest ROI set for the B-mode image. Alternatively, the slope a, the intercept b, and the intensity c may be allocated to R(red), G(green), and B(blue) of each pixel in the region of interest ROI, respectively, thereby to generate color feature image data. Alternatively, color image data in which the slope a, the intercept b and the intensity c are allocated to R(red), G(green), and B(blue) of each pixel in the region of interest (ROI), respectively, and the B-mode image data may be mixed at a predetermined ratio thereby to generate feature image data.

In step S24, the data selection unit 92 determines whether record data meeting the following condition is stored in the examination data storage unit 82. At first, the data selection unit 92 searches the examination data of the examinations past made on the same patient as the patient 150 under examination based on the patient identification information. At this time, when a plurality of examinations past made on the patient 150 are present, an examination with the latest date is selected.

Subsequently, the data selection unit 92 selects record data in which the position information is closest to the current relative position information of the ultrasound probe 2 based on the current relative position information of the ultrasound probe 2 with reference to the position information of each item of record data included in the selected examination data. For example, when the record data D(P1) to D(P4) for the observation points P1 to P4 is stored in the past examination data as illustrated in FIG. 3, if the ultrasound probe 2 is located on the upper part of the esophagus 151 as illustrated in FIG. 5, the position of the observation point P1 is closest to the ultrasound probe 2 and thus the record data D(P1) is selected.

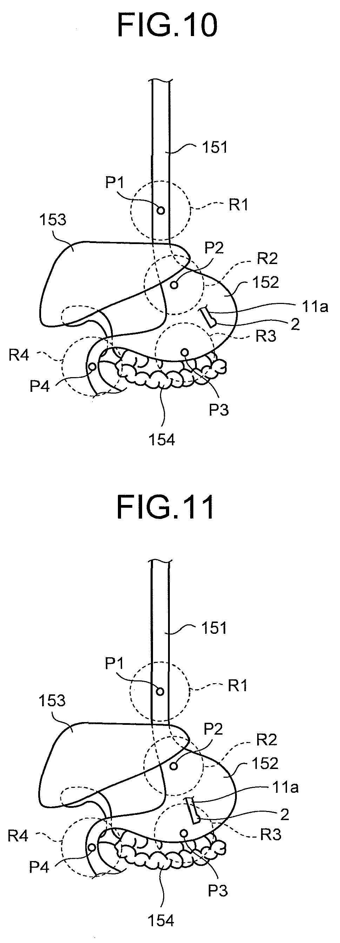

Further, the data selection unit 92 determines whether the ultrasound probe 2 is included within a display determination range in the selected record data. The display determination range is a region within a predetermined distance from an observation point, and is set for each observation point where data is recorded. For example, as illustrated in FIG. 10 and FIG. 11, the display determination ranges R1 to R4 are set for the observation points P1 to P4, respectively.

Herein, when the ultrasound probe 2 is present at the position illustrated in FIG. 10, the closest observation point to the ultrasound probe 2 is the observation point P3. At this time, however, the ultrasound probe 2 is not included within the display determination range R3 of the observation point P3. In this case, the data selection unit 92 determines that record data meeting the condition is not present (step S24: No).

On the other hand, when the ultrasound probe 2 is present at the position indicated in FIG. 11, the closest observation point to the ultrasound probe 2 is the observation point P3 and the ultrasound probe 2 is included within the display determination range R3 of the observation point P3. In this case, the data selection unit 92 determines that record data meeting the condition is present (step S24: Yes).

In step S24, when it is determined that past record data meeting the condition is not present (step S24: No), the ultrasonic observation apparatus 1 generates and displays a display screen including the feature image and the feature on the display unit 7 under control of the execution control unit 94 (step S25). Specifically, the display image data generation unit 54 generates the image data for the display screen including the frequency feature image based on the feature image data generated in step S23 and the frequency feature extracted in step S22, and the control unit 9 causes the display unit 7 to display the display screen based on the image data.

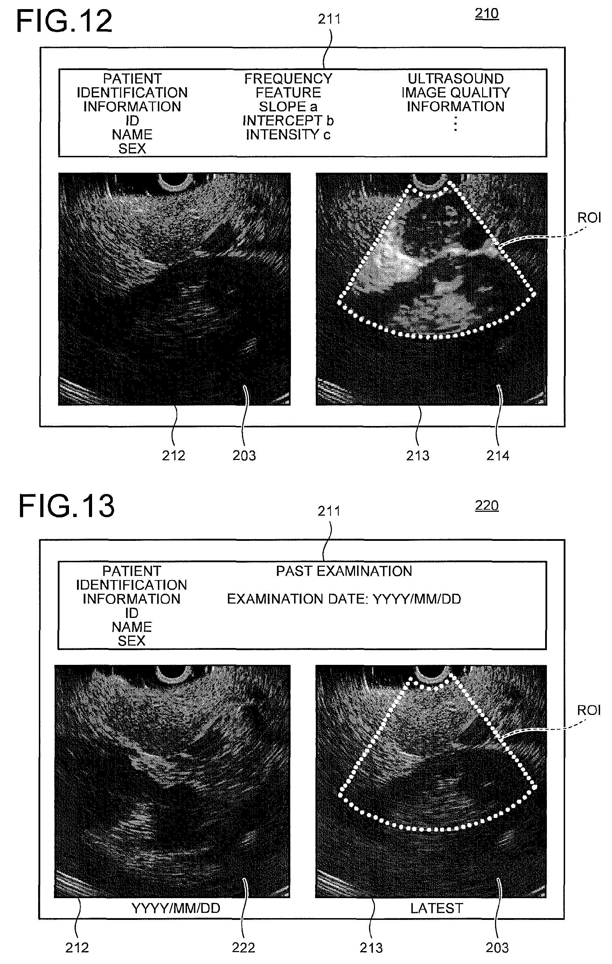

FIG. 12 is a schematic diagram illustrating an exemplary display screen displayed in step S25. A display screen 210 illustrated in FIG. 12 includes an information display region 211 displaying therein the patient identification information such as ID, name and sex, the information on the extracted frequency feature, the ultrasound image quality information such as gain and contrast, and the like, a first image display region 212, and a second image display region 213. The information on the feature may be displayed by use of an average or standard deviation of the frequency spectrum features of the FFT data groups positioned within the region of interest in addition to the features (slope a, intercept b, and intensity c).

The first image display region 212 displays therein the B-mode image 203 based on the B-mode image data generated in step S15. On the other hand, the second image display region 213 displays therein a frequency feature image 214 based on the feature image data generated in step S23. In this way, the B-mode image 203 and the frequency feature image 214 are displayed side by side so that the operator can accurately grasp the tissue characteristics in the region of interest.

In step S25, the B-mode image 203 and the frequency feature image 214 do not necessarily need to be displayed side by side, and only the frequency feature image 214 may be displayed, for example.

In subsequent step S18, when a data recording instruction signal is input (step S18: Yes), the relative position information of the ultrasound probe 2 (or the position information on the observation point), the B-mode image data, the feature image data, the frequency feature, the computation parameters used for the feature analysis, and the image processing parameters used for generating the B-mode image data and the feature image data are stored as one data set in the examination data storage unit 82 (step S19). Subsequent step S20 is as described above.

On the other hand, in step S24, when it is determined that past record data meeting the condition is present (step S24: Yes), the ultrasonic observation apparatus 1 selects and acquires the past record data and displays a screen including the past B-mode image based on the B-mode image data included in the record data and the B-mode image under real-time observation on the display unit 7 under control of the execution control unit 94 (step S26). The operator is notified of the fact that past record data meeting the condition is present, via the screen display, and the operator can recognize that an observation point capable of being compared with the past data is present near the current observation point. That is, according to the first embodiment, the display unit 7 also functions as a notification unit for giving notice to the operator the fact that past record data meeting the condition is present.

FIG. 13 is a schematic diagram illustrating exemplary display of a screen in step S26. In a display screen 220 illustrated in FIG. 13, the information display region 211 displays therein information (such as examination date) for specifying an examination for which the past record data meeting the condition is acquired in addition to the patient identification information such as ID, name and sex.

The first image display region 212 displays therein a B-mode image 222 based on the B-mode image data included in the selected past record data. On the other hand, the second image display region 213 displays therein a real-time B-mode image 203 based on the B-mode image data generated in step S15. At this time, the image processing unit 5 acquires the image processing parameters (such as gain, contrast and $\gamma$ correction coefficient) from the past record data, and may regenerate the real-time B-mode image by use of the image processing parameters.

The operator adjusts the position of the ultrasound probe 2 with reference to the past B-mode image 222 displayed on the display screen 220, thereby matching the specimen captured in the B-more image 203 under real-time observation with the specimen captured in the past B-mode image 222. Accordingly, the past B-mode image 222 and the real-time B-mode image 203 can be accurately compared with each other.

In step S27, the execution control unit 94 determines whether an image freezing instruction signal is input from the input unit 6. When an image freezing instruction signal is not input (step S27: No), the operation of the ultrasonic observation apparatus 1 proceeds to step S18.

On the other hand, when an image freezing instruction signal is input (step S27: Yes), the execution control unit 94 acquires the computation parameters and the image processing parameters from the selected past examination data with the instruction signal as a trigger (step S28), and causes the computation unit 4 and the image processing unit 5 to re-perform the processings on the acoustic ray data acquired in step S14 by use of the parameters (steps S29 to S31).

Specifically, in step S29, the frequency analysis unit 41 performs the feature analysis again on the acoustic ray data acquired in step S14 by use of the computation parameters acquired in step S28. The details of the feature analysis processing are the same as those in step S21.

In step S30, the feature extraction unit 42 re-extracts the feature from the acoustic ray data based on the analysis result acquired in the re-made feature analysis. The feature extraction processing is the same as that in step S22.

In step S31, the image processing unit 5 regenerates the feature image data based on the feature re-extracted in step S30 by use of the image processing parameters acquired in step S28. The feature image data generation processing is the same as that in step S23. At this time, the image processing unit 5 may regenerate the B-mode image data by use of the image processing parameters.

In subsequent step S32, the comparative image data generation unit 53 acquires past feature image data from the selected past record data, and generates differential image data between the past feature image data and the feature image data regenerated in step S31. The differential image data indicates a temporal change between the past examination of the specimen and the latest examination at the observation point.

In step S33, the display image data generation unit 54 generates image data of a graph or table indicating a comparison between the past feature (the frequency feature in the first embodiment) included in the selected past record data and the latest feature (same as above) re-extracted in step S30.

In step S34, the ultrasonic observation apparatus 1 generates and displays a display screen including the differential image based on the differential image data generated in step S32 and the graph or table generated in step S33 on the display unit 7.

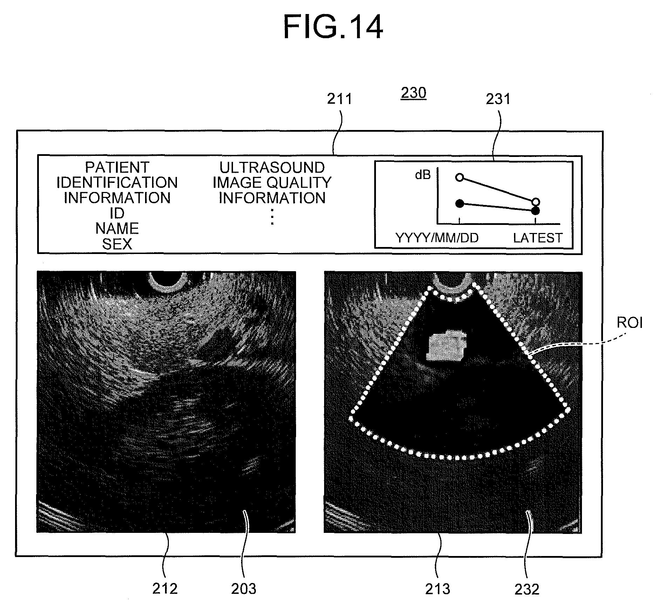

FIG. 14 is a schematic diagram illustrating exemplary display of the display screen generated in step S34. In a display screen 230 illustrated in FIG. 14, the information display region 211 displays therein a graph 231 indicating a comparison between the frequency feature extracted in the past examination and the frequency feature extracted in the latest examination in addition to the patient identification information and the ultrasound image quality information. A table indicating a comparison between the frequency features in text may be displayed instead of the graph 231.

The first image display region 212 displays therein the B-mode image 203 based on the B-mode image data generated in step S15. When the B-mode image data is also regenerated in step S31, the B-mode image based on the regenerated B-mode image data is displayed. On the other hand, the second image display region 213 displays therein a combined image 232 in which the B-mode image data and the differential image data generated in step S32 are mixed at a predetermined ratio. Alternatively, there may be generated a combined image in which the region of interest ROI set for the B-mode image is replaced with the differential image. The operator can directly and accurately grasp a temporal change in the specimen in the region of interest ROI with reference to the display screen 230.

In subsequent step S18, when a data recording instruction signal is input (step S18: Yes), the relative position information of the ultrasound probe 2 (or the position information on the observation point), the B-mode image data, the regenerated feature image data, the differential image data, the re-extracted frequency feature, the comparison result (graph or table) of the frequency feature, the computation parameters used for re-executing the feature analysis, and the image processing parameters used for regenerating the B-mode image data and the feature image data are stored as one data set in the examination data storage unit 82 (step S19). Subsequent step S20 is as described above.

As described above, according to the first embodiment, when the observation is made at an observation point identical or close to the observation point where data is recorded in a past examination, the frequency analysis is made, the feature is extracted, and the image is generated by use of the same parameters as those in the past examination, and thus the features and the images of the specimen at the observation point can be accurately compared between the past examination and the latest examination. The screen indicating a comparison between the features or the images is displayed so that the user can directly grasp a temporal change in the feature of the specimen between the past examination and the latest examination.

(First Modification)

A first modification of the first embodiment will be described below.

In the first embodiment, the parameters used for a past examination are used to perform the processings on acoustic ray data acquired in real-time (refer to steps S29 to S31). However, to the contrary, past record data may be re-processed by use of the parameters used for the real-time processings (refer to steps S21 to S23). Also in this case, the feature analysis and the image processing are performed on the past data and the latest data by use of the same parameters, and thus both of them can be accurately compared.

(Second Modification)

A second modification of the first embodiment of the present invention will be described below.

In the first embodiment, an instruction signal input from the input unit 6 is used as a trigger (refer to step S27) to perform the feature analysis again (refer to step S29) and to regenerate the feature image (refer to step S31). However, there may be configured such that when it is determined that past record data meeting the condition is present (refer to step S24), the re-processings are automatically started.

(Third Modification)

A third modification of the first embodiment of the present invention will be described below.

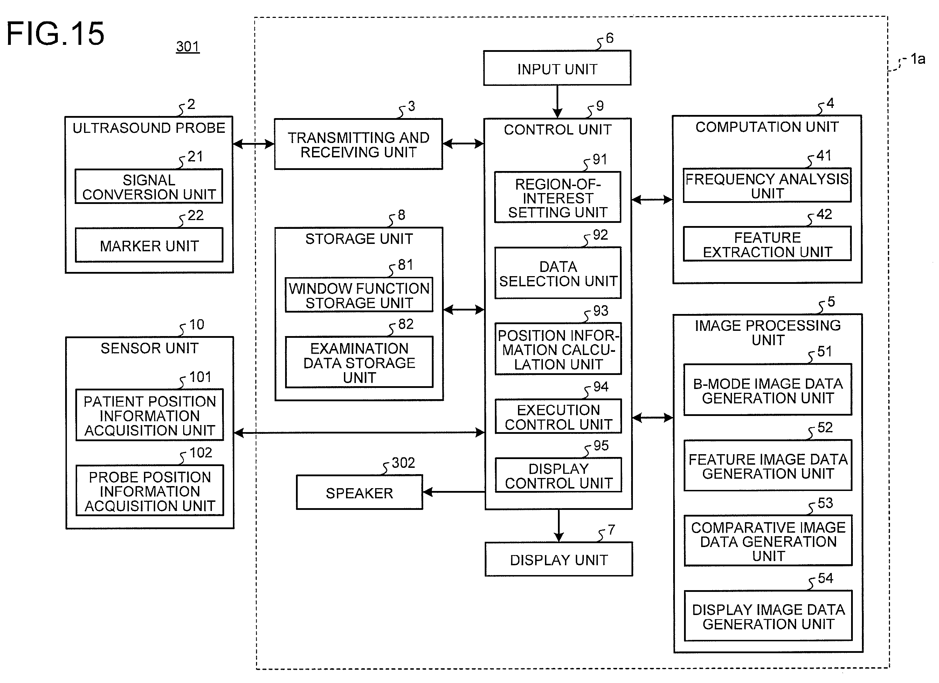

In the first embodiment, when it is determined that past record data meeting the condition is present (refer to step S24), the B-mode image at the past point of time and the B-mode image displayed in real-time are displayed on the display unit 7 to give notice that the past record data is present to the operator. However, the notification method is not limited thereto, and for example, a message that "past record data is present near ultrasound probe" may be displayed in text on the display unit 7, or a similar message may be issued by voice, or a notification sound may be issued. When the notification is made by voice or notification sound, a speaker 302 for issuing voice or notification sound under control of the control unit 9 may be provided as a notification unit as in an ultrasonic observation apparatus 301 illustrated in FIG. 15.

In this way, if the notification is made by text message, voice or the like, the execution control unit 94 causes the display unit 6 to display the screen including the past B-mode image and the real-time B-mode image (see FIG. 13) when an instruction signal for displaying the past B-mode image is input from the input unit 6. Accordingly, the operator can match the specimen captured in the real-time B-mode image with the specimen captured in the past B-mode image with reference to the past B-mode image at a desired timing.

(Fourth Modification)

A fourth modification of the first embodiment of the present invention will be described below.

The way to display a comparison result between the feature of past record data and the feature calculated for the latest examination or a differential image is not limited to the display screen 230 illustrated in FIG. 14, and various ways to display may be employed. For example, the feature image based on the feature in past record data and the feature image based on the re-extracted feature may be displayed side by side. Alternatively, the B-mode image in past record data and the real-time B-mode image regenerated by use of the image processing parameters in the past record data may be displayed side by side.

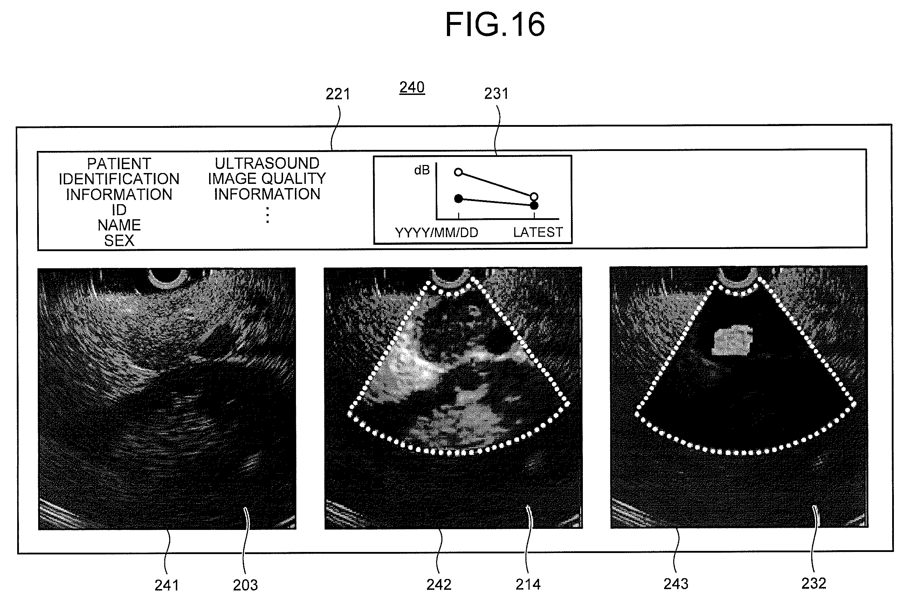

As another example, as illustrated in FIG. 16, three images may be displayed side by side. A display screen 240 illustrated in FIG. 16 includes the graph 231 indicating a comparison of the patient identification information, the ultrasound image quality information, and the frequency feature, and three image display regions 241 to 243.

The first image display region 241 displays therein the B-mode image 203 based on the B-mode image data generated in step S15. The second image display region 242 displays therein the frequency feature image 214 based on the feature image data generated in step S23. Further, the third image display region 243 displays therein the combined image 232 in which the differential image is overlapped on the B-mode image.

(Fifth Modification)

A fifth modification of the first embodiment of the present invention will be described below.

When the present invention is applied to an external ultrasound probe, various structures can be applied to the probe position information acquisition unit 102 in addition to a magnetic sensor. For example, the probe position information acquisition unit 102 may include two optical cameras to detect a position or posture (angle relative to the patient, or the like) of the ultrasound probe based on the images capturing the ultrasound probe therein. In this case, the patient position information acquisition unit 101 and the probe position information acquisition unit 102 may be formed of a common optical camera. Alternatively, a gravity sensor may be provided for the ultrasound probe to detect a posture of the ultrasound probe.

(Sixth Modification)

A sixth modification of the first embodiment of the present invention will be described below.

When extracting the frequency feature in step S22 or S30 indicated in FIG. 4, the attenuation correction may be made prior to the regression analysis of a frequency spectrum.

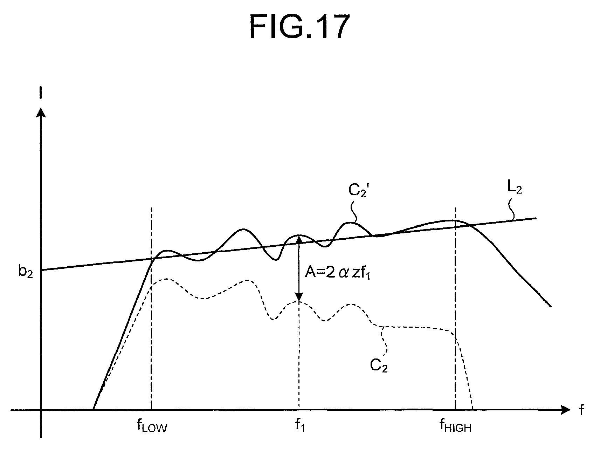

FIG. 17 is a schematic diagram for explaining the attenuation correction method according to the seventh modification. When the frequency spectrum curve $C_2$ indicated in FIG. 17 is acquired in step S21 or S29, for example, the feature extraction unit 42 corrects all the frequencies f to add the attenuation amount A in Equation (1) to the intensity I, thereby acquiring a new frequency spectrum curve $C_2'$. It is therefore possible to acquire a frequency spectrum with a reduced contribution of attenuation associated with propagation of an ultrasonic wave.

Thereafter, the feature extraction unit 42 performs the regression analysis on all the attenuation-corrected frequency spectra to extract the features of the frequency spectra. Specifically, the feature extraction unit 42 calculates the slope a, the intercept b, and the intensity c at the center frequency $f_{MID}$ in the primary expression by the regression

analysis. The line $L_2$ indicated in FIG. 17 is a regression line (intercept $b_2$) acquired by performing the feature extraction processing on the frequency spectrum curve $C_2$.

Also with the correction method, the operator can more accurately grasp the tissue characteristics of the specimen expressed in the frequency feature image.

(Second Embodiment)

A second embodiment according to the present invention will be described below.

Various well-known analysis methods can be applied to the feature analysis made in step S21 or S29 indicated in FIG. 4 in addition to the frequency analysis. There will be described in the second embodiment a case in which the contrast analysis is applied for an ultrasonic echo acquired in the contrast harmonic echo (CHE) method.

The CHE method is a technique in which a contrast agent such as microbubbles is introduced into the body of a patient, and a harmonic signal generated by irradiating the contrast agent with an ultrasonic wave is extracted and made into an image to acquire bloodstream information. Refer to Japanese Laid-open Patent Publication No. 2010-259672 for the details of the CHE method, for example.

In order to perform the CHE method, the computation unit 4 performs the contrast analysis on acoustic ray data output from the transmitting and receiving unit 3 in step S21 or S29 indicated in FIG. 4. More specifically, two ultrasonic signals, which are offset from each other in phase by 180°, are successively transmitted from the ultrasound probe 2 and the ultrasonic echoes thereof are received, respectively, thereby to generate acoustic ray data, and the computation unit 4 adds the acoustic ray data to generate a signal with the offset fundamental wave component and the emphasized second-order harmonic component.

Alternatively, two ultrasonic signals with the same phase and the amplitudes of 1 : n are successively transmitted from the ultrasound probe 2 and the ultrasonic echoes thereof are received, respectively, thereby to generate acoustic ray data, and the computation unit 4 multiplies either item of acoustic ray data by n and subtracts the n-times acoustic ray data from the other acoustic ray data to generate a signal with the offset fundamental wave component and the emphasized second-order harmonic component.

Alternatively, an ultrasonic signal is transmitted from the ultrasound probe 2 once and the ultrasonic echo thereof is received to generate acoustic ray data, and the computation unit 4 may perform high-pass filter processing on the acoustic ray data to extract the harmonic component.

Further, in this case, the computation unit 4 performs an envelope detection processing on the harmonic-component signal and extracts an amplitude of the envelope as the feature in step S22 or S30.

In this case, the feature image data generation unit 52 uniformly allocates the feature (the amplitude of the envelope) extracted by the computation unit 4 to R(red), G(green) and B(blue) of each pixel in the region of interest ROI set for the B-mode image in step S23 or S31, thereby generating CHE image data.

(Third Embodiment)

A third embodiment according to the present invention will be described below.

The elastic analysis may be made on an ultrasonic echo acquired by the ultrasonic elastography method for the feature analysis made in steps S21 and S29 indicated in FIG. 4. The ultrasonic elastography method is called tissue elastic imaging, and is a technique in which an ultrasound probe is contacted and pressed on the body surface of a patient and a distribution of displacements (distortions) of a body tissue caused when the body tissue is pressed is expressed in an image thereby to visualize hardness of the body tissue. As a body tissue is harder, its deformation is more difficult to cause and its displacement is smaller, and as a body tissue is more flexible, its displacement is larger. Refer to Japanese Laid-open Patent Publication No. 2007-105400 publication for the details of the ultrasonic elastography method, for example.

When the ultrasonic elastography method is performed, the computation unit 4 performs the elastic analysis on the acoustic ray data output from the transmitting and receiving unit 3 in step S21 or S29 indicated in FIG. 4. More specifically, the computation unit 4 accumulates the acoustic ray data output from the transmitting and receiving unit 3 per frame and performs the 1D or 2D correlation processing on the latest frame data (acoustic ray data for one frame) and frame data predetermined time before the latest frame data to measure a displacement or motion vector (direction and magnitude of displacement) at each point on a cross-sectional image.

In this case, in step S22 or S30, the computation unit 4 extracts a magnitude of displacement or motion vector at each point on the cross-sectional image as the feature (the distortion amount).

In this case, in step S23 or S31, the feature image data generation unit 52 performs the image processing such as the smoothing processing in a coordinate space, the contrast optimization processing, or the smoothing processing in an inter-frame temporal axis direction on the distortion amount at each point on the cross-sectional image extracted by the computation unit 4. Then, a pixel value (luminance) depending on the distortion amount after the image processing is uniformly allocated to R(red), G(green), and B(blue) of each pixel in the region of interest ROI set for the B-mode image, thereby generating gray scale elastic image data. Specifically, as the distortion amount is larger, the luminance is set to be higher. Alternatively, a pixel value allocated to each color is changed depending on the distortion amount to generate color elastic image data. Specifically, the allocation amount of R(red) is larger for a pixel with the larger distortion amount, and the allocation amount of B(blue) is larger for a pixel with the smaller distortion amount.

The first to third embodiments according to the present invention, and the modifications have been described above, but the present invention is not limited to the first to third embodiments and the modifications, and various inventions can be formed by combining a plurality of components disclosed in each embodiment or modification as needed. For example, some components may be excluded from all the components demonstrated in each embodiment or modification for formation, or components demonstrated in different embodiments or modifications may be combined as needed for formation.

Reference Signs List

| | |
|---|---|
| 1, 301 | Ultrasonic observation apparatus |
| 2 | Ultrasound probe |
| 3 | Transmitting and receiving unit |
| 4 | Computation unit |
| 5 | Image processing unit |
| 6 | Input unit |
| 7 | Display unit |
| 8 | Storage unit |
| 9 | Control unit |
| 10 | Sensor unit |
| 11 | Ultrasonic endoscope |
| 21 | Signal conversion unit |
| 22 | Marker unit |
| 41 | Frequency analysis unit |
| 42 | Feature extraction unit |
| 51 | B-mode image data generation unit |
| 52 | Feature image data generation unit |
| 53 | Comparative image data generation unit |
| 54 | Display image data generation unit |
| 81 | Window function storage unit |
| 82 | Examination data storage unit |
| 91 | Region-of-interest setting unit |
| 92 | Data selection unit |
| 93 | Position information calculation unit |
| 94 | Execution control unit |
| 95 | Display control unit |
| 101 | Patient position information acquisition unit |
| 102 | Probe position information acquisition unit |
| 150 | Patient |
| 302 | Speaker |

**Claims**

1. An ultrasonic observation apparatus for transmitting an ultrasonic wave toward a specimen and receiving the ultrasonic wave reflected from the specimen by an ultrasound probe to generate an image based on the received ultrasonic wave, the ultrasonic observation apparatus comprising:

a computation unit configured to extract a feature of the received ultrasonic wave;
an image processing unit configured to generate image data based on the received ultrasonic wave;
a position information acquisition unit configured to acquire relative position information indicating a relative position of the ultrasound probe relative to a patient as an examination target;

a storage unit configured to store a plurality of data sets including: position information on an observation point; the feature extracted by the computation unit for the observation point; the image data generated by the image processing unit for the observation point; and parameters used for extracting the feature and generating the image data such that the plurality of data sets associates with information for identifying the patient;

a data selection unit configured to search the plurality of data sets based on the relative position information acquired by the position information acquisition unit, and to select a data set meeting a predetermined condition; and

an execution control unit configured to, when the data selection unit selects the data set meeting the predetermined condition, cause the computation unit to re-extract one of a feature included in the data set selected by the data selection unit and a latest feature extracted by the computation unit by use of the parameters used for extracting the other of the features.

2. The ultrasonic observation apparatus according to claim 1, wherein the predetermined condition indicates that a position of the observation point presented by the position information included in the data set is closest to the relative position of the ultrasound probe relative to the patient and within a predetermined range of the relative position.

3. The ultrasonic observation apparatus according to claim 1 or 2, further comprising a notification unit configured to, when the data selection unit selects the data set meeting the predetermined condition, give notice that the data set is selected.

4. The ultrasonic observation apparatus according to claim 3, wherein the notification unit is a display unit configured to display on a screen an image based on the image data included in the data set selected by the data selection unit.

5. The ultrasonic observation apparatus according to claim 3, wherein the notification unit is configured to give notice that the data set is selected, by voice, notification sound or text display.

6. The ultrasonic observation apparatus according to any one of claims 3 to 5, further comprising an input unit configured to input a signal depending on an operation from outside into the execution control unit, wherein the execution control unit is configured to cause the computation unit to start re-extracting the one of the features depending on a predetermined signal input from the input unit after giving notice that the data set is selected.

7. The ultrasonic observation apparatus according to claim 1 or 2, wherein the execution control unit is configured to cause the computation unit to start re-extracting the one of the features when the data selection unit selects the data set meeting the predetermined condition.

8. The ultrasonic observation apparatus according to claim 1 or 2, wherein the image processing unit is configured to generate feature image data based on the feature, wherein when the one of the features is re-extracted, the image processing unit is configured to generate feature image data based on the one of the features re-extracted by use of the parameters used for generating feature image data based on the other of the features.

9. The ultrasonic observation apparatus according to claim 8, wherein the image processing unit is configured to further generate differential image data indicating a difference between first feature image data generated based on the one of the features re-extracted and second feature image data generated based on the other of the features.

10. The ultrasonic observation apparatus according to claim 9, wherein the image processing unit is configured to generate B-mode image data based on the received ultrasonic wave, wherein when the differential image data is generated, the image processing unit is configured to further generate combined image data in which the differential image data and the B-mode image data are combined.

11. The ultrasonic observation apparatus according to claim 10, wherein the image processing unit is configured to further generate display image data indicating a screen including: at least one of a B-mode image based on the B-mode image data and a feature image based on the feature image data; and a combined image based on the combined image data.

12. The ultrasonic observation apparatus according to claim 1 or 2, wherein when the one of the features is re-extracted, the image processing unit is configured to further generate image data indicating a graph or table of a comparison between the one of the features re-extracted and the other of the features.

13. The ultrasonic observation apparatus according to any one of claims 1 to 12, wherein
the position information acquisition unit comprises:

a probe position information acquisition unit configured to acquire position information indicating a position of the ultrasound probe;
a patient position information acquisition unit configured to acquire position information indicating a position of the patient; and
a position information calculation unit configured to calculate a relative position coordinate of the ultrasound probe relative to the patient, based on the position information of the ultrasound probe and the position information of the patient.

14. The ultrasonic observation apparatus according to claim 13, wherein
the probe position information acquisition unit comprises:

a first marker provided on the ultrasound probe; and
a sensor unit configured to detect the first marker and to output a detection signal, and

the patient position information acquisition unit comprises:

a second marker configured to be mounted on a body surface of the patient; and
an optical camera configured to image the second marker to generate an image.

15. The ultrasonic observation apparatus according to any one of claims 1 to 14, wherein the computation unit is configured to perform a frequency spectrum analysis on the received ultrasonic wave to calculate a frequency spectrum, and to extract the feature by use of a result of an approximation processing for the frequency spectrum.

16. The ultrasonic observation apparatus according to any one of claims 1 to 14, wherein the computation unit is configured to extract a harmonic signal from the received ultrasonic wave, and to extract an amplitude of an envelope of the harmonic signal as the feature.

17. The ultrasonic observation apparatus according to any one of claims 1 to 14, wherein the computation unit is configured to measure a distortion amount in the specimen based on the received ultrasonic wave, and to extract the distortion amount as the feature.

18. A method for operating an ultrasonic observation apparatus for transmitting an ultrasonic wave toward a specimen and receiving the ultrasonic wave reflected from the specimen by an ultrasound probe to generate an image based on the received ultrasonic wave, the method comprising:

a computation step of extracting, by a computation unit, a feature of the received ultrasonic wave;
an image processing step of generating, by an image processing unit, image data based on the received ultrasonic wave;
a position information acquisition step of acquiring, by a position information acquisition unit, relative position information indicating a relative position of the ultrasound probe relative to a patient as an examination target;
a data selection step of searching, by a data selection unit, a plurality of data sets stored in a storage unit based on the relative position information acquired in the position information acquisition step, and selecting a data set meeting a predetermined condition, the storage unit storing the plurality of data sets including: position information on an observation point; the feature extracted by the computation unit for the observation point; the image data generated by the image processing unit for the observation point; and parameters used for extracting the feature and generating the image data such that the plurality of data sets associates with information for identifying the patient; and
an execution control step of, when the data set meeting the predetermined condition is selected, causing, by an execution control unit, the computation unit to re-extract one of a feature included in the data set selected by the data selection unit and a latest feature extracted by the computation unit by use of the parameters used for extracting the other of the features.

19. A program for operating an ultrasonic observation apparatus for transmitting an ultrasonic wave toward a specimen and receiving the ultrasonic wave reflected from the specimen by an ultrasound probe to generate an image based on the received ultrasonic wave, the program causing the ultrasonic observation apparatus to execute:

a computation step of extracting, by a computation unit, a feature of the received ultrasonic wave;

an image processing step of generating, by an image processing unit, image data based on the received ultrasonic wave;

a position information acquisition step of acquiring, by a position information acquisition unit, relative position information indicating a relative position of the ultrasound probe relative to a patient as an examination target;

a data selection step of searching, by a data selection unit, a plurality of data sets stored in a storage unit based on the relative position information acquired in the position information acquisition step, and selecting a data set meeting a predetermined condition, the storage unit storing the plurality of data sets including: position information on an observation point; the feature extracted by the computation unit for the observation point; the image data generated by the image processing unit for the observation point; and parameters used for extracting the feature and generating the image data such that the plurality of data sets associates with information for identifying the patient; and

an execution control step of, when the data set meeting the predetermined condition is selected, causing, by an execution control unit, the computation unit to re-extract one of a feature included in the data set selected by the data selection unit and a latest feature extracted by the computation unit by use of the parameters used for extracting the other of the features.

# FIG.1

1

**INPUT UNIT** — 6

**ULTRASOUND PROBE** — 2
- **SIGNAL CONVERSION UNIT** — 21
- **MARKER UNIT** — 22

**TRANSMITTING AND RECEIVING UNIT** — 3

**CONTROL UNIT** — 9
- **REGION-OF-INTEREST SETTING UNIT** — 91
- **DATA SELECTION UNIT** — 92
- **POSITION INFORMATION CALCULATION UNIT** — 93
- **EXECUTION CONTROL UNIT** — 94
- **DISPLAY CONTROL UNIT** — 95

**COMPUTATION UNIT** — 4
- **FREQUENCY ANALYSIS UNIT** — 41
- **FEATURE EXTRACTION UNIT** — 42

**STORAGE UNIT** — 8
- **WINDOW FUNCTION STORAGE UNIT** — 81
- **EXAMINATION DATA STORAGE UNIT** — 82

**SENSOR UNIT** — 10
- **PATIENT POSITION INFORMATION ACQUISITION UNIT** — 101
- **PROBE POSITION INFORMATION ACQUISITION UNIT** — 102

**IMAGE PROCESSING UNIT** — 5
- **B-MODE IMAGE DATA GENERATION UNIT** — 51
- **FEATURE IMAGE DATA GENERATION UNIT** — 52
- **COMPARATIVE IMAGE DATA GENERATION UNIT** — 53
- **DISPLAY IMAGE DATA GENERATION UNIT** — 54

**DISPLAY UNIT** — 7

1a

# FIG.2

# FIG.3

PATIENT ID, PATIENT NAME

EXAMINATION ID, EXAMINATION TIME AND DATE

D(P1)  D(P2)  D(P3)  D(P4)

RECORD DATA

POSITION INFORMATION
B-MODE IMAGE DATA
FEATURE IMAGE DATA
DIFFERENTIAL IMAGE DATA
FREQUENCY FEATURE
COMPUTATION PARAMETER
IMAGE PROCESSING
PARAMETER
COMPARISON RESULT
BETWEEN FEATURES

FIG.4

START

ACQUIRE REFERENCE POSITION INFORMATION — S10

ACQUIRE PATIENT IDENTIFICATION INFORMATION — S11

S12
FREEZE IS RELEASED? — NO

YES

MEASURE POSITION INFORMATION — S13

MEASURE NEW SPECIMEN — S14

GENERATE B-MODE IMAGE DATA — S15

S16
REGION OF INTEREST IS SET? — NO

YES

S17
DISPLAY B-MODE IMAGE

FEATURE ANALYSIS — S21

EXTRACT FEATURE — S22

GENERATE FEATURE IMAGE DATA — S23

S24
RECORD DATA MEETING CONDITION IS PRESENT? — NO

YES

DISPLAY PAST IMAGE — S26

S27
FREEZE? — NO

YES

ACQUIRE PARAMETER — S28

RE-EXECUTE FEATURE ANALYSIS — S29

RE-EXTRACT FEATURE — S30

REGENERATE FEATURE IMAGE DATA — S31

GENERATE DIFFERENTIAL IMAGE DATA — S32

GENERATE GRAPH OR TABLE INDICATING COMPARISON BETWEEN FEATURES — S33

DISPLAY SCREEN INCLUDING DIFFERENTIAL IMAGE AND TABLE OR GRAPH OF COMPARISON BETWEEN FEATURES — S34

S25
DISPLAY SCREEN INCLUDING FEATURE IMAGE AND FEATURE

S18
RECORD? — NO

YES — S19
STORE DATA SET

S20
END? — NO

YES

END

# FIG.5

# FIG.6

PATIENT
IDENTIFICATION
INFORMATION
ID
NAME
SEX

201     202   203

# FIG.7

```
                    ┌──────────────────────┐
                    │   FEATURE ANALYSIS    │
                    └──────────────────────┘
                               │
                    ┌──────────────────────┐
                    │       k=k₀            │ ～S211
                    └──────────────────────┘
                               │
                    ┌──────────────────────┐
                    │    Z^(k)=Z^(k)₀       │ ～S212
                    └──────────────────────┘
                               │
                    ┌──────────────────────┐
                    │ ACQUIRE FFT DATA GROUP│ ～S213
                    └──────────────────────┘
                               │
                    ┌──────────────────────┐
                    │ APPLY WINDOW FUNCTION │ ～S214
                    └──────────────────────┘
                               │
                          S215 ◇
                    ╱  FFT DATA GROUP  ╲  NO      ┌───────────────────────────┐
                    ╲   IS NORMAL?     ╱─────────▶│ INSERT ZERO DATA CORRE-    │ ～S216
                          ◇                       │ SPONDING TO SHORTFALL     │
                         YES                      └───────────────────────────┘
                               │
                    ┌──────────────────────┐
                    │PERFORM FFT COMPUTATION│ ～S217
                    └──────────────────────┘
                               │
                    ┌──────────────────────┐
                    │    Z^(k)=Z^(k)+D      │ ～S218
                    └──────────────────────┘
                               │
                          S219 ◇
                    ╱  Z^(k)>Z^(k)_max? ╲  NO
                    ╲                   ╱────────▶
                          ◇
                         YES
                               │
                    ┌──────────────────────┐
                    │       k=k+1          │ ～S220
                    └──────────────────────┘
                               │
                          S221 ◇
                  NO ╱   k>k_max?    ╲
                    ╲                ╱
                          ◇
                         YES
                               │
                    ┌──────────────────────┐
                    │       RETURN          │
                    └──────────────────────┘
```

# FIG.8

$Z^{(k)}_0$

$D$

$SR_k$

$Z^{(k)}_{max}$

$F_1$

$F_2$

$F_3$

$F_{K-1}$

$F_K$

# FIG.9

$I$

$L_1'$

$b_0$

$C_1$

$L_1$

$f_{LOW}$

$f_{HIGH}$

$f$

# FIG.10

# FIG.11

## FIG.12

211   210

| PATIENT IDENTIFICATION INFORMATION ID NAME SEX | FREQUENCY FEATURE SLOPE a INTERCEPT b INTENSITY c | ULTRASOUND IMAGE QUALITY INFORMATION ⋮ |

ROI

212   203   213   214

## FIG.13

211   220

| PATIENT IDENTIFICATION INFORMATION ID NAME SEX | PAST EXAMINATION EXAMINATION DATE: YYYY/MM/DD |

ROI

YYYY/MM/DD   LATEST

212   222   213   203

# FIG.14

211    230    231

PATIENT
IDENTIFICATION
INFORMATION
ID
NAME
SEX

ULTRASOUND
IMAGE QUALITY
INFORMATION
⋮

dB

YYYY/MM/DD    LATEST

ROI

212    203    213    232

# FIG.15

EP 3 078 330 A1

# FIG.17

## INTERNATIONAL SEARCH REPORT

| | International application No. |
|---|---|
| | PCT/JP2014/079163 |

**A. CLASSIFICATION OF SUBJECT MATTER**
*A61B8/08*(2006.01)i

According to International Patent Classification (IPC) or to both national classification and IPC

**B. FIELDS SEARCHED**

Minimum documentation searched (classification system followed by classification symbols)
A61B8/08

Documentation searched other than minimum documentation to the extent that such documents are included in the fields searched

| Jitsuyo Shinan Koho | 1922–1996 | Jitsuyo Shinan Toroku Koho | 1996–2014 |
|---|---|---|---|
| Kokai Jitsuyo Shinan Koho | 1971–2014 | Toroku Jitsuyo Shinan Koho | 1994–2014 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 4677199 B2 (Hitachi Medical Corp.), 27 April 2011 (27.04.2011), entire text; all drawings & JP 2005-296436 A | 1-19 |
| A | WO 2012/063929 A1 (Olympus Medical Systems Corp.), 18 May 2012 (18.05.2012), entire text; all drawings & US 2013/0030296 A1 & JP 5307939 B2 & EP 2599441 A1 | 1-19 |
| A | JP 2005-323629 A (GE Medical Systems Global Technology Company, L.L.C.), 24 November 2005 (24.11.2005), entire text; all drawings (Family: none) | 1-19 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 03 December 2014 (03.12.14) | 16 December 2014 (16.12.14) |

| Name and mailing address of the ISA/ | Authorized officer |
|---|---|
| Japan Patent Office | |
| Facsimile No. | Telephone No. |

Form PCT/ISA/210 (second sheet) (July 2009)

**EP 3 078 330 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008006169 A
- JP 2005323629 A
- JP 2010259672 A
- JP 2007105400 A